# EUROPÄISCHE PATENTANMELDUNG

(11) **EP 1 563 854 A1**
(43) Veröffentlichungstag der Anmeldung: **17.08.2005**
(21) Anmeldenummer: 05000434.0
(22) Anmeldetag: 12.01.2005
(51) Int. Cl.: A61L 2/26

(54) **Medizinischer Sterilisierbehälter mit Gasaustauschfilter**

(30) Priorität: 12.02.2004 DE 202004002095 U
(71) Anmelder: Gottfried Storz, Medizintechnik GmbH & Co. KG, 78532 Tuttlingen (DE)
(72) Erfinder: Koch, Gernod, 78589 Dürbheim (DE); Kreidler, Winfried, 78532 Tuttlingen (DE)
(74) Vertreter: Neymeyer, Franz, Dipl.-Ing. (FH)

(57) **Zusammenfassung**

Medizinischer Sterilisierbehälter mit einem abnehmbaren Deckel (1), der in einem durchbrochenen Gasaustauschbereich (3) eine Filtereinheit für den Gasaustausch aufweist. Innerhalb eines umlaufenden Rahmenelements (6), ist ein Filterblatt (5) zwischen zwei mit Durchbrüchen (4) versehenen Auflageflächen auswechselbar angeordnet. Ein Halter (15) ist durch mehrere Renkverbindungselemente (10, 41) lösbar mit dem Rahmenelement (6) verbunden. In einer Ringnut (35) ist ein elastischer Dichtungsring (50) angeordnet, der den durchbrochenen Gasaustauschbereich (3) des Deckels (1) gegen das Rahmenelement (6) abdichtet. Das Rahmenelement (6) ist in Form eines dünnwandigen, runden Ringes fest und dicht mit der Innenfläche (11) des Deckels (1) verbunden und mit mehreren gleichmäßig verteilt angeordneten Renkverschlußbolzen (10) versehen. Der scheibenförmige Halter (15) ist auf der Innenseite eines haubenartig ausgebildeten, mehrfach durchbrochenen Griffteils (26) befestigt, welches im Randbereich des Halters (15) innerhalb eines mit den Renkverschlußbolzen (10) verbindbaren Kupplungsflansches (40) eine Ringnut (35) mit dem Dichtungsring (50) aufweist.

## Beschreibung

Die Erfindung betrifft einen medizinischen Sterilisierbehälter mit abnehmbarem Deckel, der in wenigstens einem durchbrochenen Gasaustauschbereich eine Filtereinheit für den Gasaustausch aufweist, bei der innerhalb eines umlaufenden, auf der Innenseite des Deckels befestigten Rahmenelements, ein Filterblatt zwischen zwei im Flächenbereich des Filterblattes mit Durchbrüchen versehenen, im wesentlichen ebenen Auflageflächen auswechselbar angeordnet ist, wobei die eine Auflagefläche zugleich Anpreßfläche eines scheibenförmigen Halters ist, der durch mehrere Renkverbindungselemente lösbar mit dem Rahmenelement verbunden ist und wobei in einer Ringnut eines gegen die Innenfläche des Deckels pressbaren Elements ein elastischer Dichtungsring angeordnet ist, der den durchbrochenen Gasaustauschbereich des Deckels gegen das Rahmenelement abdichtet.

Sterilisierbehälter der gattungsgemäßen Art sind in unterschiedlichen Variationen bekannt. Sie bestehen gewöhnlich aus einem Behälterunterteil und aus einem aufklappbaren Behälterdeckel. Sie werden dazu benutzt, beispielsweise chirurgische Instrumente bzw. Bestecke und anderes Sterilisiergut in einem Heißdampf erzeugenden Sterilisierungsschrank zu sterilisieren.

Dabei befindet sich das Sterilisiergut in dem dicht geschlossenen Sterilisierbehälter, dessen Innenraum nur über die Filtereinheit mit der Außenumgebung derart in Verbindung steht, dass der Sterilisierungsdampf in das Behälterinnere eindringen kann und dass nach dem Sterilisierungsvorgang in- oder außerhalb des Sterilisierungsschrankes über die Filtereinheit wieder ein Luftaustausch während der Erkaltungsphase stattfinden kann. Dabei muß jedoch sichergestellt sein, dass keine erneute Kontamination der sterilisierten Instrumente bzw. des sonstigen Sterilisiergutes innerhalb des Sterilisierbehälters mehr stattfinden kann, d.h., dass der Gasaustausch nur durch das Filterblatt hindurch erfolgen kann.

Wichtig dabei ist auch, dass sich das Filterblatt auf einfache Weise austauschen läßt und dass es im eingebauten Zustand nicht der Gefahr ausgesetzt ist, durch dünne spitze Instrumententeile beschädigt zu werden.

Bei den bekannten Sterilisierbehältern, bei denen das Filterblatt unmittelbar an der Innenseite des Behälterdeckels angelegt ist und die in einem vorzugsweise runden Flächenbereich angeordneten Durchbrüche des Deckels innenseitig abdeckt, ist der scheibenförmige Halter, der zum Halten des Filterblattes an der Innenseite des Dekkels vorgesehen ist, durch einen zentralen Verbindungszapfen, der in eine Verriegelungshülse eines zentralen, erhabenen Griffteils des Halters angeordnet ist, mit dem Deckel lösbar verbunden.

Als Dichtungsmittel sind in einer äußeren Randnut des Halters und im Innenbereich eines den Verbindungszapfen überdeckenden und umschließenden Griffteils jeweils elastische, ringförmige Dichtungen untergebracht, die unmittelbar auf der Innenfläche des Behälterdeckels aufliegen. Dabei ist der Anpreßdruck insbesondere des in der äußeren Ringnut liegenden Dichtungsringes relativ gering, so dass schon bei geringen Unebenheiten in der Anpreßfläche Dichtungslecks entstehen können. Solche Sterilisierbehälter sind schon seit längerer Zeit im Handel und auch im Gebrauch (siehe z. B. Firmenschrift "Sterilcontainer-Systeme" der Firma Aesculap, Tuttlingen bzw. der Firma Erbrich und Firmenschrift "Steriset Sterilcontainer der Firma Wagner, München).

Auch aus DE 198 32 823 C1 ist ein solcher Sterilisierbehälter mit einer tellerförmigen Filterhalterung bekannt, die einen äußeren Andruckrand und eine zentrale Befestigungseinrichtung mit einem am Behälterdeckel befestigten Kupplungszapfen aufweist.

Aus DE 202 03 984 U1 ist aber auch bereits ein Sterilbehälter bekannt, bei dem der Filterhalter mittels Renkverbindungselementen an einem Rahmenteil lösbar befestigt ist, wobei sich diese Renkverbindungselemente radial außerhalb des Filterblattbereiches befinden. Im Innern des Rahmenteils ist eine Filterauflage angeordnet, die mit dem Rahmenteil ebenfalls durch Renkverbindungselemente lösbar verbunden ist. Diese Filterauflage besteht aus einem runden Ring, der in seinem Zentrum einen durch mehrere radial verlaufende Speichen mit ihm verbundenen Zentrierzapfen für den Filterhalter aufweist. Dabei ist das Rahmenteil, in dem das Filterblatt mittels der beiden genannten zusätzlichen Elemente gelagert ist, an der Innenseite des Behälterdeckels axial federnd befestigt, so dass sich der Rahmen insgesamt von der Innenseite des Deckels abheben kann, wenn im Sterilisierbehälter Unterdruck herrscht, damit der Druckausgleich nicht nur durch das Filter hindurch und somit schneller erfolgen kann.

Bei diesem Sterilbehälter ist nur der Ring der Filterauflage mit einem, diesen gegen den Deckel abdichtenden Dichtungsring versehen, der allerdings nicht auf der ebenen Innenfläche des Behälterdeckels, sondern auf einer abgeflachten Ringrippe des Deckels aufliegt.

Der Erfindung liegt die Aufgabe zugrunde, einen Sterilisierbehälter der eingangs genannten Art zu schaffen, bei dem die Filtereinheit aus wenigen, einfach handhabbaren Teilen besteht, die eine sichere und vollständige Abdichtung des Filterblattrandes gegen das ungefilterte Eindringen von Außenluft gewährleistet und durch welche zugleich sicher gestellt ist, dass das vorwiegend aus chirurgischen Instrumenten bestehende Sterilsiergut mit dem Filterblatt nicht schädlich in Berührung kommen kann.

Gelöst wird diese Aufgabe erfindungsgemäß dadurch, dass das Rahmenelement (6) in Form eines dünnwandigen, runden Ringes fest und dicht mit der Innenfläche (11) des Dekkels (1) verbunden und mit mehreren gleichmäßig verteilt angeordneten Renkverschlußbolzen (10) versehen ist und dass der scheibenförmige Halter (15) auf der Innenseite eines haubenartig ausgebildeten, mehrfach durchbrochenen Griffteils (26) befestigt ist, welches im Randbereich des Halters (15) innerhalb eines mit den Renkverschlußbolzen (10) verbindbaren Kupplungsflansches (40) eine Ringnut (35) mit dem Dichtungsring (50) aufweist.

Eine so gestaltete Filtereinheit ist nicht nur einfach und kostengünstig herstellbar, sondern sie ist auch einfach handhabbar und sie gewährleistet vor allem ein sicheres und zuverlässiges Abdichten der Anlegefläche des Filterblattes auf der Innenseite des Deckels gegenüber dem Innenraum des Sterilisierbehälters. Dabei ist besonders wichtig, dass der aus einem gummiähnlichen, d.h. elastischen Material bestehende Dichtungsring, der in der Ringnut der Filtereinheit untergebracht ist, dichtend gegen eine Innenfläche des als Ring ausgebildeten Rahmenelementes gepreßt wird, wenn der Kupplungsflansch durch die Renkverbindungselemente mit dem Rahmenelement verbunden ist.

Grundsätzlich besteht die Möglichkeit, das Rahmenelement unmittelbar an der Innenseite des Deckels anzuformen, z.B. wenn der Deckel als Gußteil, Spritzgußteil oder Druckgußteil hergestellt wird.

Bei der Ausgestaltung nach Anspruch 2 kann die dichtende Verbindung zwischen dem Rahmenelement und der Innenfläche des Deckels beispielsweise durch eine dünne, flache Dichtungseinlage zwischen dem Rahmenelement und dem Deckel oder aber durch eine nachträglich angebrachte Fugendichtung aus Silikon oder ähnlichem Dichtungsmaterial hergestellt werden.

Eine besonders günstige Ausgestaltung des Rahmenelementes zur Erzielung der gewünschten zuverlässigen Abdichtung ist Gegenstand des Anspruches 3. Das Rahmenelement kann gemäß Anspruch 4 ein L- oder U-förmiges Querschnittsprofil aufweisen, wobei ein U-förmiges Querschnittsprofil dem Rahmenelement eine höhere Biegestabilität verleiht.

Durch die Ausgestaltung nach Anspruch 5 erhält auch der Kupplungsflansch des abnehmbaren Teils der Filtereinheit eine ausreichende Stabilität gegen Verbiegung, so dass auch der für eine zuverlässige Abdichtung erforderliche Anpreßdruck für den Dichtungsring gewährleistet werden kann.

Während es grundsätzlich auch möglich wäre, das Rahmenelement am Deckel mittels Schrauben zu befestigen, hat die Ausgestaltung nach Anspruch 6 den Vorteil einer höheren und nicht veränderbaren Verbindungsstabilität und einfacheren Herstellung einer stabilen Verbindung zwischen dem Rahmenelement und dem Deckel.

Während die Ausgestaltungen nach den Ansprüchen 7 und 8 sowohl handhabungstechnische als auch herstellungstechnische Vorteile bieten, besteht der Vorteil der Ausgestaltung nach Anspruch 9 darin, dass die den Dichtungsring aufnehmende Ringnut sich unmittelbar an den Kupplungsflansch anschließt und somit die vom Kupplungsflansch auf den Dichtungsring auszuübenden Anpreßkräfte auf dem kürzesten Wege übertragen werden und so die größtmögliche Wirksamkeit gewährleisten.

Auch die Ausgestaltung nach Anspruch 10 dient der Erzielung eines ausreichenden Anpreßdruckes im Randbereich des Filterblattes, wobei dieser Anpreßdruck aber mehr der Lagefixierung des Filterblattes als der Abdichtung dient, die bei der erfindungsgemäßen Anordnung hauptsächlich von dem Dichtungsring gewährleistet wird.

Während die Ausgestaltung nach Anspruch 11 hinsichtlich der Erzielung eines ausreichenden Anpreßdruckes auf den Randbereich des Filterblattes dem Ausgleich von Maßtoleranzen, insbesondere der Dicke des Filterblattes, dient, kann mit der Ausgestaltung nach Anspruch 12 der Vorteil erreicht werden, dass der Halter als relativ dünne Blechscheibe aus Edelstahl ausgebildet sein kann.

Durch die Ausgestaltung nach Anspruch 13 wird nicht nur eine umlaufende Abdichtung der von dem Filterblatt abgekehrten Außenseite des Halterrandes durch den Dichtungsring gewährleistet, sondern auch ein zusätzliches Anpressen des Filterblattrandes an die Innenfläche des Deckels, wodurch nicht nur ein zusätzlicher Dichtungseffekt, sondern auch eine zusätzliche Lagefixierung des Filterblattes erfolgt.

Die gemäß Anspruch 14 vorgesehene Formgebung des Griffteils ermöglicht eine einfache und zweckmäßige Handhabung und stellt zugleich sicher, daß sich im Behälter befindenden Instrumente mit dem verletzlichen Filterblatt nicht in Berührung kommen können.

Durch diese Ausgestaltungen des Querschnittsprofils des Dichtungsrings gemäß den Ansprüchen 15 und 16 ergeben sich bei wenigstens gleich guter Dichtungsqualität wesentlich geringere Reibungskräfte zwischen dem drehbeweglichen Griffteil und dem Deckel bzw. dem Rahmenelement, so daß sich der Griffteil leichter drehen läßt.

Anhand der Zeichnung wird im folgenden ein Ausführungsbeispiel der Erfindung näher erläutert. Es zeigt:
- Fig. 1: in isometrischer Perspektivdarstellung einen mit einer Filtereinheit ausgestatteten Behälterdeckel in Rückenlage;
- Fig. 2: die Bestandteile der Filtereinheit mit dem Dekkel aus Fig. 1 in isometrischer Explosionsdarstellung;
- Fig. 3: den mit der Filtereinheit versehenen Deckel der Fig. 1 in Draufsicht;
- Fig. 4: einen Schnitt IV-IV aus Fig. 3;
- Fig. 5: die rechte Hälfte der Fig. 4 in vergrößertem Maßstab;
- Fig. 6: einen Ausschnitt VI aus Fig. 5 in weiter vergrößertem Maßstab;
- Fig. 6a: in gleicher Darstellung wie Fig. 6 eine andere Ausführungsform des Rahmenprofils und mit einem im Durchmesser kleineren Filterblatt mit entsprechend veränderter Anpreßlage des Dichtungsringes;
- Fig. 6b: in leichter Vergrößerung die gleichen Teile wie Fig. 6a, jedoch mit einem anderen Querschnittsprofil des Dichtungsrings und
- Fig. 6c: die gleiche Darstellung wie Fig. 6b, jedoch mit einem anderen Querschnittsprofil des Dichtungsrings.

Sterilisierbehälter der hier in Frage stehenden Art werden dazu benutzt, chirurgische Bestecke, Instrumente und dgl. aufzunehmen und in einem Sterilisierschrank zu sterilisieren sowie dazu, diese chirurgischen Bestecke und Instrumente nach dem Sterilisieren im sterilisierten Zustand aufzubewahren, bis sie wieder in Benutzung genommen werden.

Damit während des Sterilisiervorganges zwischen dem Innenraum des Sterilisierbehälters und dem Innenraum des Sterilisierschrankes ein Gasaustausch, insbesondere ein Heißdampfaustausch, stattfinden kann, ist der in den Zeichnungsfiguren dargestellte Deckel 1 eines dazugehörigen, aber hier nicht dargestellten, kastenartigen Behälterunterteils auf seiner Innenseite mit einer Filtereinheit 2 versehen.

Diese Filtereinheit 2 ist auf der Innenseite des Deckels 1 angeordnet, um einen mehrfach durchbrochenen Gasaustauschbereich 3 abzudecken. Dieser Gasaustauschbereich 3 ist mit einer Vielzahl von Bohrungen 4 versehen, die innenseitig durch ein Filterblatt 5 abgedeckt sind.

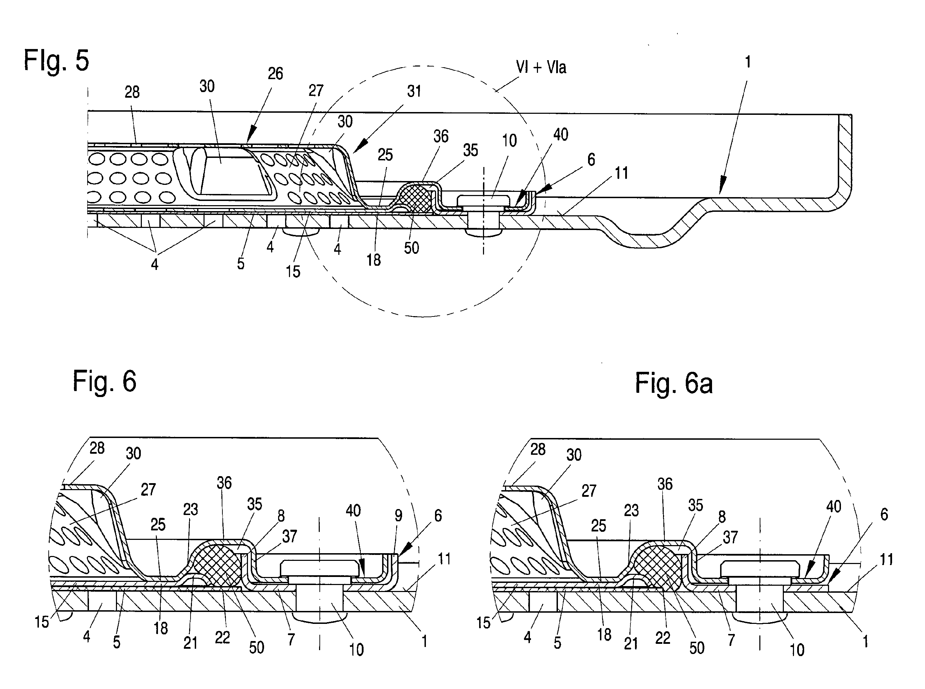

Die Filtereinheit 2 besteht aus einem Rahmenelement 6, das die Form eines dünnwandigen Ringes mit einem U-förmigen oder nach der Ausführungsform gemäß Fig. 6a mit einem L-förmigen Querschnittsprofil aufweist. Vorzugsweise ist dieses Rahmenelement 6 als tiefgezogenes Blechteil aus Edelstahl ausgebildet.

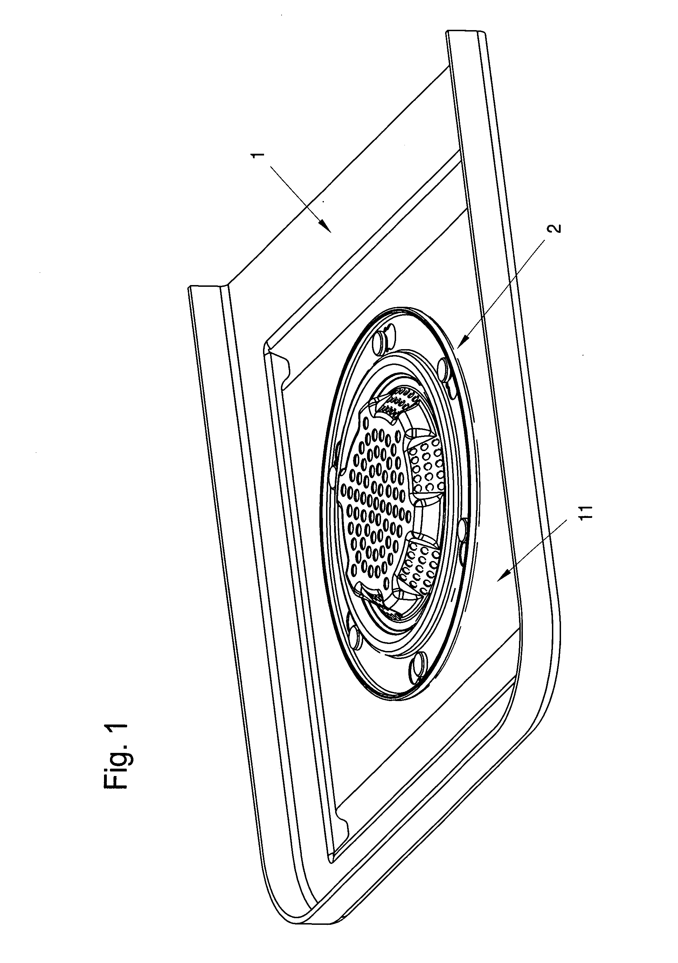

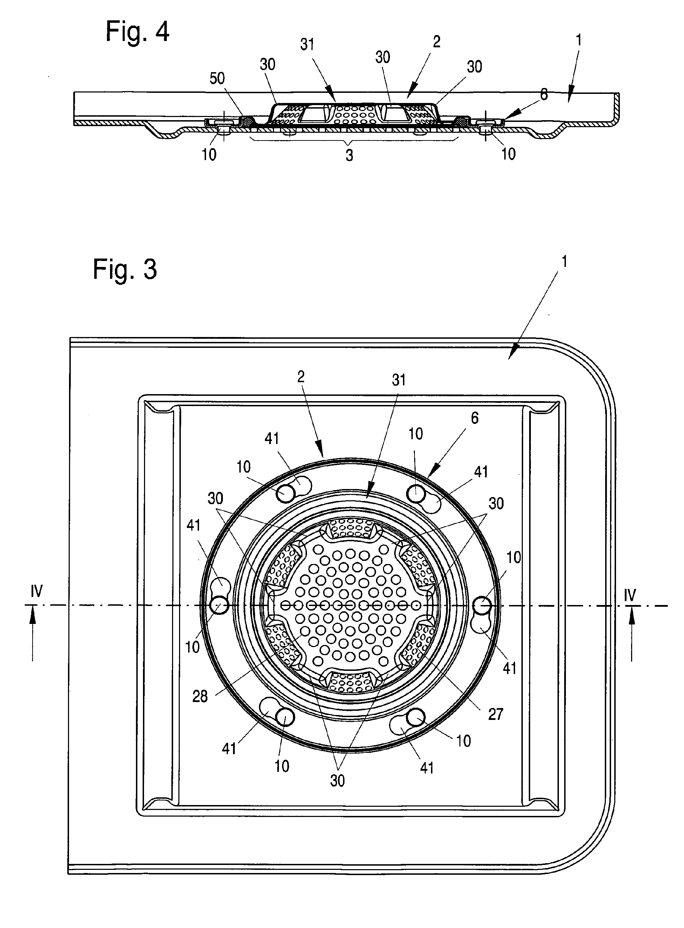

Bei der Ausführungsform der Fig. 1 bis 6 besitzt dieses Rahmenelement 6 an einem ringförmigen Flachteil 7 eine innere, rechtwinklig angewinkelte Ringwand 8 und eine äußere, ebenfalls rechtwinklig angewinkelte Ringwand 9, die das U-Profil ergeben.

Mit insgesamt sechs in Umfangsrichtung gleichmäßig verteilt angeordneten Renkverschlußbolzen 10 ist das Rahmenelement 6 auf einer planebenen Innenfläche 11 des Deckels 1 so befestigt und angeordnet, dass es den durchbrochenen Gasaustauschbereich 3 konzentrisch und dicht umschließt. Dabei ist zwischen dieser Innenfläche 11 und dem Flachteil 7 des Rahmenelementes 6 eine abdichtende Silikonschicht od.dgl. angeordnet. Statt dieser plattgepreßten Silikonschicht könnte auch eine klebende Ringdichtung in der inneren und/oder äußeren Randfuge des Rahmenelementes 6 vorgesehen sein.

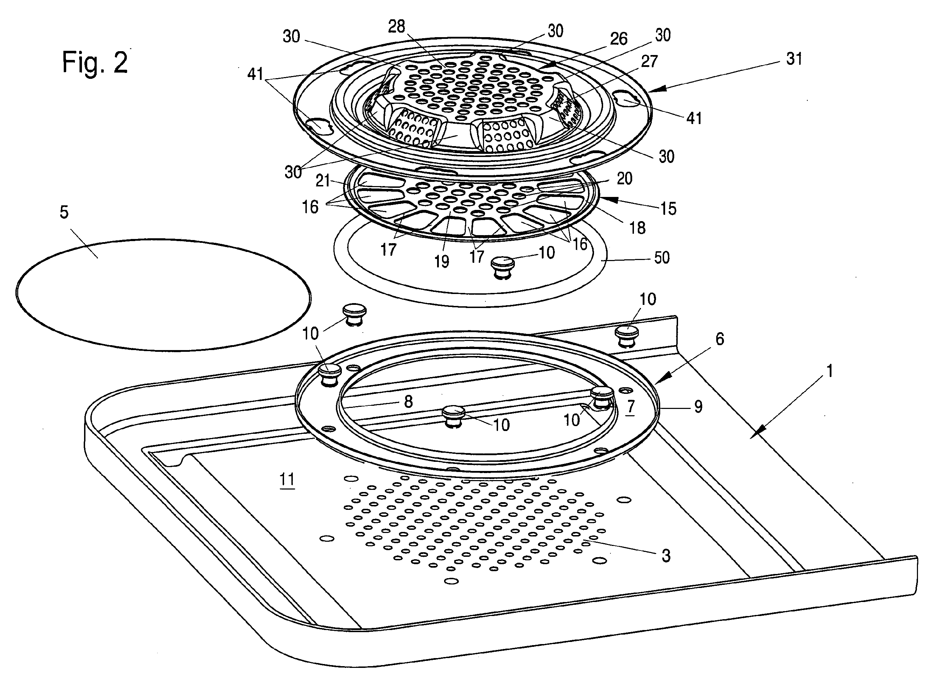

Das aus geeignetem Filtermaterial bestehende Filterblatt 5, das die Form einer runden Scheibe aufweist und dessen Durchmesser auf den Innendurchmesser des Rahmenelements 6 abgestimmt ist, wird von einem scheibenförmigen Halter 15 zumindest leicht gegen die Innenfläche 11 des Deckels 1 gedrückt und dort fixiert. Dieser Halter 15 ist, wie die Fig. 2 am besten zeigt, mit einer Vielzahl von trapezartigen Durchbrüchen 16 versehen, die durch Radialstege 17 voneinander getrennt sind. Dabei verbinden diese Radialstege 17 einen äußeren flachen Ringbund 18 mit einem inneren, runden und in der gleichen Ebene liegenden Plattenabschnitt 19, der eine Vielzahl von Bohrungen 20 aufweist. Durch diese Bohrungen und die Durchbrüche 16 sowie durch die Bohrungen 4 des Deckels 1 kann über das Filterblatt ein Gasaustausch zwischen der Außenseite des Dekkels 1 und dessen Innenseite bzw. dem Innenraum des Sterilisierungsbehälters stattfinden, wenn der Deckel 1 geschlossen ist.

An den flachen Ringbund 18 des Halters 15 schließt sich ein umlaufendes, gewölbtes Randprofil 21 an.

Mit dem genannten Ringbund 18 ist der Halter 15 auf geeignete Weise, vorzugsweise durch Schweißen oder durch Nieten, an einem flachen Druckring 25 eines haubenartigen Griffteils 26 unlösbar befestigt. Dieser haubenartige Griffteil 26 besteht aus einem durch Tiefziehen geformten Blechteil aus Edelstahl. Wie aus den Zeichnungen unschwer erkennbar ist, weist der Griffteil 26 eine im wesentlichen kegelförmige Stützwand 27 auf, deren Abschluß eine planebene Bodenwand 28 bildet. Sowohl die Stützwand 27 als auch die Bodenwand 28 sind jeweils mit einer Vielzahl von Bohrungen 29 versehen, durch welche der Gasaustausch zum Filterblatt 5 stattfinden kann.

Dieser haubenförmige Griffteil 26 schützt das Filterblatt 5 gegen Beschädigungen, die durch die im Sterilisierungsbehälter liegenden Instrumente, insbesondere im Bereich der Durchbrüche 16 des Halters 15 hervorgerufen werden könnten.

Um den Griffteil 26 leicht und sicher halten und drehen zu können, ist die Stützwand 27 mit mehreren radial nach außen überstehenden, höckerartigen Griffprofilen 30 versehen, die in Umfangsrichtung gleichmäßig verteilt angeordnet sind. Mit diesen Griffprofilen 30 bildet der Griffteil 26 das kegelstumpfförmige Zentralteil eines gemeinsam mit dem Halter 15 vom Deckel 1 bzw. von dem Rahmenelement 6 abnehmbaren Befestigungs- und Schutzelementes 31 für das Filterblatt, dessen Randbereich umlaufend zuverlässig und sicher gegen den Innenraum des Sterilisierbehälters bzw. gegen die Innenfläche 11 des Deckels 1 abgedichtet sein muß, wenn die Filtereinheit geschlossen ist.

Um dies zu ermöglichen, ist in einer Ringnut 35, ein Dichtungsring 50 auf besondere Weise angeordnet. Diese Ringnut 35 ist von einem im wesentlichen U-förmigen Ringprofil 36 gebildet, das sich aus der Ebene des Druckringes 25 erhebt und den Kupplungsflansch 40 umschließt. Dabei ist ein Profilabschnitt 37 zugleich Teil des U-förmig profilierten Kupplungsflansches 40. Dieser Kupplungsflansch 40 ist so profiliert, dass er in den ebenfalls U- oder L-förmigen Ringkörper des Rahmenelementes 6 eingelegt werden kann. Der U-förmig profilierte Kupplungsflansch 40 ist somit einstückig über das die Ringnut 35 bildende Ringprofil 36 mit dem Druckring 25 und über diesen auch mit dem Griffteil 26 verbunden.

Dabei steht der Druckring 25 über einen schräg zur Halterebene verlaufenden und somit axial elastischen Wandabschnitt 23 mit dem die Ringnut 35 bildenden Ringprofil 36 in direkter Verbindung.

Damit das Befestigungs- und Schutzteil 31 zusammen mit dem daran befestigten Halter 15 mit Hilfe der Renkverschlußbolzen 10 am Rahmenelement 6 festsitzend, jedoch wieder lösbar, befestigt werden kann, ist sein Kupplungsflansch 40 mit zu den Renkverschlußbolzen passenden Renkverschlußschlitzen 41 versehen.

Das U-förmige Querschnittsprofil des Kupplungsflansches 40 trägt zur Erhöhung der Biegefestigkeit bei.

In der Ringnut 35, die unmittelbar innerhalb des Kupplungsflansches 40 liegt, ist der aus elastischem Material bestehende Dichtungsring 50 gelagert. Wenn der Kupplungsflansch 40 mit den Renkverschlußbolzen 10 in Eingriff gebracht ist, liegt der Dichtungsring 50 dichtend an der Innenseite der inneren Ringwand 8 des Rahmenelementes 6 an. Zugleich liegt er auch mit einer gewissen axialen Andruckkraft auf dem das Randprofil 21 des Halters 15 radial überragenden Randabschnitt 22 des Filterblattes 5 dichtend auf. Dabei stützt sich dieser Dichtungsring 50 auch an dem Randprofil 21 dichtend ab. Bei Verwendung eines Filterblattes 15, dessen Durchmesser kleiner ist als der Innendurchmesser der Ringwand 8, kann sich der Dichtungsring 50, wie in Fig. 6a dargestellt ist, gleichzeitig auch auf der Innenfläche 11 des Deckels 1 dichtend abstützen.

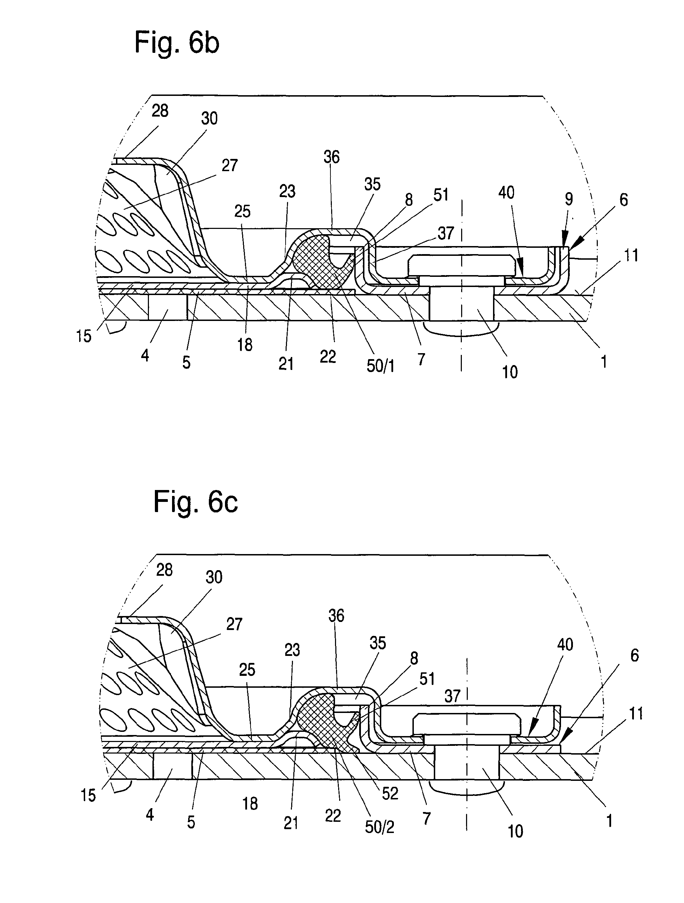

Es ist aus den Fig. 5, 6 und 6a ohne weiteres ersichtlich, dass zu dem geschilderten Zweck, nämlich zur Erreichung einer zuverlässigen Abdichtung des Filterblattrandes, die innere Ringwand 8 des Rahmenelementes 6 in die Ringnut 35 hineinragt, in welcher der Dichtungsring 50 gelagert ist. Im entspannten Zustand, d.h. wenn die Renkverbindung zwischen dem Kupplungsflansch 40 und dem Rahmenelement 6 gelöst und der Dichtungsring 50 keinem axialen Preßdruck ausgesetzt ist, hat dieser einen Außendurchmesser, der etwas kleiner ist als der Innendurchmesser der Ringwand 8, so dass der Dichtungsring 50 leicht in das Rahmenelement 6 eingeführt werden kann. Erst beim Schließen der Renkverbindung ergibt sich ein entsprechender axialer Druck, der die Verformung des Dichtungsringes 50 in der Weise mit sich bringt, dass dieser sowohl an der Innenfläche der Ringwand 8 als auch auf dem Randabschnitt 22 des Filterblattes 5 bzw. auf der Innenfläche 11 des Deckels dichtend anliegt.

Um dies zu erreichen, ist die Renkverbindung zwischen dem Kupplungsflansch 40 und den Renkverschlußbolzen 10 mit einer Keilwirkung ausgestattet.

Die Funktionsweise der Renkverbindungselemente 10 und 41 sind der Fachwelt hinlänglich bekannt, so dass eine Erläuterung an dieser Stelle nicht erforderlich ist.

Bei Verwendung von Filterblättern, deren Durchmesser kleiner ist als der Innendurchmesser der Ringwand 8 des Rahmenelementes 6, ist es zweckmäßig, im Zentrum des Gasaustauschbereichs in an sich bekannter Weise einen Zentrierzapfen vorzusehen, der von einem zentralen Loch des Filterblattes zentrierend aufgenommen werden kann.

Wie aus den Fig. 5, 6 und 6a erkennbar ist, besteht zwischen der Ringwand 8 des Rahmenelementes 6 und dem Randprofil 21 des Halters 15 ein Ringspalt, der von einem Teil des Dichtungsringes 50 ausgefüllt wird. Innerhalb dieses Ringspaltes liegt der Dichtungsring 50, bei der Ausführungsform der Fig. 5 und 6, auf dem das Randprofil 21 des Halters 15 radial nach außen überragenden Randabschnitt 22 des Filterblattes 15 auf.

Bei der Ausführungsform der Fig. 6a liegt der Dichtungsring 50 sowohl auf dem kleineren Randabschnitt 22 des Filterblattes 5 als auch auf der Innenfläche 11 des Dekkels auf, und zwar in einem Bereich, der sich radial unmittelbar an den Randabschnitt 22 des Filterblattes 5 anschließt.

In jedem Fall ist aber sichergestellt, dass der Dichtungsring 50 ein Eindringen kontaminierter Luft oder sonstiger Gase in das Innere des Sterilisierbehälters sicher und zuverlässig verhindert, wenn das Befestigungs- und Schutzteil 31 in der dargestellten Weise durch die Renkverbindungselemente mit dem Deckel 1 verbunden, d.h., wenn die Filtereinheit ordnungsgemäß installiert ist.

Durch die räumliche Nähe des Kupplungsflansches 40 zum Dichtungsring 50 bzw. zum Randabschnitt 22 des Filterblattes 5 ist auch sichergestellt, dass die zur Erzielung einer Abdichtung und die entsprechende Verformung des Dichtungsringes benötigten Anpreßkräfte unmittelbar von den Renkverbindungselementen 10/41 über den Kupplungsflansch 40 auf den Dichtungsring 50 übertragen werden. Wichtig dabei ist auch, dass der Dichtungsring 50, der auch bei abgenommenem Befestigungs- und Schutzteil auf dem Randprofil 21 des Halters 15 mit einer gewissen Vorspannung selbsthaltend und dichtend aufliegt, auch dichtend an der metallenen Innenfläche der Ringwand 8 zur Anlage kommt, wenn die Filtereinheit geschlossen ist. Es sind somit beste Dichtungsverhältnisse gewährleistet.

In Verbindung mit den auch zwischen dem Flachteil 7 des Rahmenelementes 2 und der Innenfläche 11 des Deckels 1 vorgesehenen Dichtungsmitteln, die in der Zeichnung nicht dargestellt sind, ist die angestrebte vollkommene Abdichtung des Behälterinnenraumes gegen das Eindringen kontaminierter Luft oder anderer Gase am Filterblatt 5 vorbei völlig ausgeschlossen.

Durch die besonders vorteilhafte Anordnung des Dichtungsringes und dessen Zusammenwirken mit der Ringwand 8 des Rahmenelementes 6, ist es für das Erreichen der gewünschten Abdichtung belanglos, ob auch der Kupplungsflansch 40 dicht schließend auf dem Flachteil 7 des U- oder L-förmig profilierten Rahmenelementes 6 aufliegt.

Auch das zum Austauschen des Filterblattes 5 erforderliche Abnehmen des Befestigungs- und Schutzteils 31 läßt sich durch die an sich bekannten Renkverbindungselemente 10 und 41 leicht bewerkstelligen.

In der Praxis hat sich gezeigt, daß sich der Griffteil 26 relativ zum Rahmenelement 6 nur schwer, d.h. nur unter Aufwendung eines großen Drehmoments, drehen läßt, wenn der Dichtungsring 50 vollvolumig ausgebildet ist und großflächig und/oder mit großer Anpreßkraft an der Ringwand 8 bzw. auf der Innenfläche 11 des Deckels 1 anliegt. Um diesen Nachteil zu vermeiden und trotzdem eine gute Abdichtungsqualität zu gewährleisten, ist es empfehlenswert, den Dichtungsring 50/1, wie in Fig. 6b dargestellt, mit einem Querschnittsprofil zu versehen, das eine umlaufende, an Ringwand 8 des Rahmenelements 6 dichtend anliegende Dichtungslippe 51 aufweist.

Dabei kann der Dichtungsring 50/2, wie in Fig. 6c dargestellt, mit einer zusätzlichen auf der Innenfläche 11 des Deckels (1) und/oder auf dem Randabschnitt 22 des Filterblatts 5 aufliegenden Dichtungslippe 52 versehen sein.

Durch diese Ausgestaltungen des Querschnittsprofils des Dichtungsrings 50/1 bzw. 50/2 ergeben sich bei wenigstens, gleich guter Dichtungsqualität wesentlich geringere Reibungskräfte zwischen dem drehbeweglichen Griffteil 26 und dem Deckel 1 bzw. dem Rahmenelement 6, so daß sich der Griffteil 26 leichter drehen und somit bequemer handhaben läßt.

## Patentansprüche

1. Medizinischer Sterilisierbehälter mit abnehmbarem Deckel (1), der in wenigstens einem durchbrochenen Gasaustauschbereich (3) eine Filtereinheit für den Gasaustausch aufweist, bei der innerhalb eines umlaufenden, auf der Innenseite des Deckels befestigten Rahmenelements (6), ein Filterblatt (5) zwischen zwei im Flächenbereich des Filterblattes (5) mit Durchbrüchen (4) versehenen, im wesentlichen ebenen Auflageflächen auswechselbar angeordnet ist, wobei die eine Auflagefläche zugleich Anpreßfläche eines scheibenförmigen Halters (15) ist, der durch mehrere Renkverbindungselemente (10, 41) lösbar mit dem Rahmenelement (6) verbunden ist und wobei in einer Ringnut (35) eines gegen die Innenfläche des Deckels (1) pressbaren Elements ein elastischer Dichtungsring (50) angeordnet ist, der den durchbrochenen Gasaustauschbereich (3) des Deckels (1) gegen das Rahmenelement (6) abdichtet,
**dadurch gekennzeichnet,**
**dass** das Rahmenelement (6) in Form eines dünnwandigen, runden Ringes fest und dicht mit der Innenfläche (11) des Deckels (1) verbunden und mit mehreren gleichmäßig verteilt angeordneten Renkverschlußbolzen (10) versehen ist und dass der scheibenförmige Halter (15) auf der Innenseite eines haubenartig ausgebildeten, mehrfach durchbrochenen Griffteils (26) befestigt ist, welches im Randbereich des Halters (15) innerhalb eines mit den Renkverschlußbolzen (10) verbindbaren Kupplungsflansches (40) eine Ringnut (35) mit dem Dichtungsring (50) aufweist.

2. Sterilisierbehälter nach Anspruch 1, **dadurch gekennzeichnet, dass** das Rahmenelement (6) als separates Teil ausgebildet und mit einem Flachteil (7) an der Innenfläche (11) des Deckels (1) dicht anliegend befestigt ist.

3. Sterilisierbehälter nach Anspruch 1 oder 2, **dadurch gekennzeichnet, dass** das Rahmenelement (6) eine am Flachteil (7) angewinkelte Ringwand (8) aufweist, die innerhalb der Ringnut (35) des Griffteils (26) dichtend am Dichtungsring (50) anliegt.

4. Sterilisierbehälter nach Anspruch 3 , **dadurch gekennzeichnet, dass** das Rahmenelement (6) ein L- oder U-förmiges Querschnittsprofil aufweist.

5. Sterilisierbehälter nach Anspruch 4, **dadurch gekennzeichnet, dass** der Kupplungsflansch (40) die Form eines auf dem Flachteil (7) des Rahmenelements (6) flach aufliegenden runden Ringes mit einem L- oder U-förmigen Querschnittsprofil aufweist.

6. Sterilisierbehälter nach einem der Ansprüche 1 bis 5, **dadurch gekennzeichnet, dass** das Rahmenelement (6) aus einem Blechteil besteht, das mit dem Deckel (1) vernietet oder verschweißt und mittels eines haftenden Dichtungsmittels umlaufend dichtend verbunden ist.

7. Sterilisierbehälter nach Anspruch 6, **dadurch gekennzeichnet, dass** der Kupplungsflansch (40) mit mehreren in gleichen Abständen angeordneten Renkverschlußschlitzen (41) versehen ist.

8. Sterilisierbehälter nach Anspruch 6, **dadurch gekennzeichnet, dass** das Rahmenelement (6) mittels der Renkverschlußbolzen (10) mit dem Deckel (1) vernietet ist.

9. Sterilisierbehälter nach Anspruch 1, **dadurch gekennzeichnet, dass** die Ringnut (35) von einem im Querschnitt zumindest annähernd U-förmigen, sich aus der Ebene des Kupplungsflansches (40) erhebenden Ringprofil (36) gebildet ist, von dem ein Profilabschnitt (37) zugleich Teil des Kupplungsflanschprofils (40) ist.

10. Sterilisierbehälter nach Anspruch 1, **dadurch gekennzeichnet, dass** der scheibenförmige Halter (15) mit seinem Randbereich (18) an einem radial zwischen der Ringnut (35) und dem haubenartigen Griffteil (26) liegenden flachen Druckring (25) befestigt ist.

11. Sterilisierbehälter nach Anspruch 10, **dadurch gekennzeichnet, dass** der Druckring (25) über einen schräg zur Halterebene verlaufenden und somit axial elastischen Wandabschnitt (23) mit dem die Ringnut (35) bildenden Ringprofil verbunden ist.

12. Sterilisierbehälter nach Anspruch 1, **dadurch gekennzeichnet, dass** der Druckring (25) zumindest annähernd in der Ebene des Kupplungsflansches (40) liegt.

13. Sterilisierbehälter nach Anspruch 1, **dadurch gekennzeichnet, dass** der Halter (15) mit einem teilweise unter den Dichtungsring (50) greifenden, gewölbten Randprofil (21) versehen ist, wobei der Dichtungsring (50) dichtend auf dem Randprofil (21) des Halters (15) und zugleich in einem zwischen diesem Randprofil (21) und dem Rahmenelement (6) liegenden Ringspalt auf der Innenfläche (11) des Deckels (1) und/oder auf dem Randabschnitt (22) des Filterblattes (5) dichtend aufliegt.

14. Sterilisierbehälter nach einem der Ansprüche 1 bis 12, **dadurch gekennzeichnet, dass** der Griffteil (26) aus einem kegelstumpfförmigen Zentralteil mit einer mehrfach perforierten, kegelförmigen Stützwand (27) und einem ebenfalls mehrfach perforierten flachen Boden (28) besteht, in dessen Randbereich mehrere fingergerechte Griffprofile (30) angeordnet sind.

15. Sterilisierbehälter nach Anspruch 1 oder 13, **dadurch gekennzeichnet, dass** der Dichtungsring (50/1, 50/2) mit einer umlaufenden, an Ringwand (8) des Rahmenelements (6) dichtend anliegenden Dichtungslippe (51) versehen ist.

16. Sterilisierbehälter nach Anspruch 15, **dadurch gekennzeichnet, dass** der Dichtungsring (50/2) mit einer zusätzlichen auf der Innenfläche (11) des Deckels (1) und/oder auf dem Randabschnitt (22) des Filterblatts (5) aufliegenden Dichtungslippe (52) versehen ist.
